# EUROPEAN PATENT APPLICATION

(11) **EP 4 349 244 A1**
(43) Date of publication of application: **10.04.2024**
(21) Application number: 21942443.9
(22) Date of filing: 31.05.2021
(51) Int. Cl.: A61B 5/00, A61B 1/00

(54) **INSPECTION METHOD AND SYSTEM DIRECTLY APPLYING NONINVASIVE OCT TO ENDOMETRIUM, AND DEVICE**

(30) Priority: 24.05.2021 CN 202110567731
(71) Applicant: Tomophase Limited, Wanchai, Hong Kong 999077 (HK)
(72) Inventor: WANG, Chichiu, Hong Kong 999077 (CN); ZHANG, Ruizhe, Hong Kong 999077 (CN); GENG, Ke, Hong Kong 999077 (CN); LI, Yejing, Hong Kong 999077 (CN); LI, Bailing, Hong Kong 999077 (CN); GAO, Jun, Hong Kong 999077 (CN)
(74) Representative: Zaboliene, Reda
(86) International application number: PCT/CN2021/097270
(87) International publication number: WO 2022/246872

(57) **Abstract**

Embodiments of the application relate to the technical field of Optical Coherence Tomography (OCT) devices, and disclose a detection method and system directly applying noninvasive OCT to an endometrium, and a detection device. The method includes: inserting an OCT probe into a uterine cavity through a cervical orifice until reaching a fundus of a uterus; withdrawing the OCT probe, and starting an image acquisition apparatus at the same time to obtain a plurality of OCT images; and dividing the plurality of OCT images into four areas, namely a uterine fundus area, a uterine upper section area, a uterine lower section area, and a cervical area, each area having one or more OCT images, and analyzing the OCT image of each area. By implementing the embodiments of the application, each area may be analyzed according to the OCT image, a human body cannot be injured, and the detection is accurate.

## Description

### Technical Field

The application relates to the technical field of Optical Coherence Tomography (OCT) devices, and in particular to a detection method and system directly applying noninvasive OCT to an endometrium, and a detection device.

### Background

The main existing manners for detecting an endometrium are as follows: for example, the thickness of the endometrium and a lesion in a uterine cavity may be observed through B-ultrasound; hemostasis and a pathological diagnosis may also be performed through diagnostic cucrettage; and in addition, a tissue may also be taken for biopsy through hysteroscopy.

The information obtained by the above first manner is less, if accurate information needs to be obtained, the second or third manner needs to be used, but the latter two manners result in certain injury to a human body, the detection cost is higher, and the detection period is longer.

### Summary

In view of the defects, embodiments of the application disclose a detection method and system directly applying noninvasive OCT to an endometrium, and a detection device, which may obtain endometrial images through the OCT to complete the detection of the endometrium, etc.

A first aspect of the embodiments of the application discloses a detection method directly applying noninvasive OCT to an endometrium. The method includes the following operations.

An OCT probe is inserted into a uterine cavity through a cervical orifice until reaching a fundus of a uterus.

The OCT probe is withdrawn, and an image acquisition apparatus is started at the same time to obtain a plurality of OCT images.

The plurality of OCT images are divided into four areas, namely a uterine fundus area, a uterine upper section area, a uterine lower section area, and a cervical area, each area having one or more OCT images, and the OCT image of each area is analyzed.

As an optional implementation, in the first aspect of the embodiments of the application, the operation that the OCT probe is inserted into the uterine cavity through the cervical orifice until reaching the fundus of the uterus includes the following operations.

A catheter provided with the OCT probe is connected by using a reproductive tract guide.

The reproductive tract guide is manually or automatically controlled to drive the catheter to be inserted into the uterine cavity through the cervical orifice until reaching the fundus of the uterus.

As an optional implementation, in the first aspect of the embodiments of the application, a speed of manual or automatic control is not greater than 0.3 cm/s.

As an optional implementation, in the first aspect of the embodiments of the application, the operation that the OCT probe is withdrawn, and the image acquisition apparatus is started at the same time to obtain the plurality of OCT images includes the following operations.

A power mechanism is started to drive the OCT probe to withdraw from the fundus of the uterus to the cervical orifice at a constant speed. A speed of withdrawal is 0.1 to 0.3 cm/s.

The OCT probe is withdrawn, and the image acquisition apparatus is started at the same time to acquire the plurality of OCT images in a withdrawal process at a fixed frequency.

As an optional implementation, in the first aspect of the embodiments of the application, the operation that the OCT probe is withdrawn, and the image acquisition apparatus is started at the same time to obtain the plurality of OCT images includes the following operations.

The OCT probe is driven to withdraw from the fundus of the uterus to the cervical orifice, and a position of movement of the OCT probe is acquired through a displacement sensor.

When a distance between the positions of two times of movement is equal to a set distance, the image acquisition apparatus is started to acquire the plurality of OCT images in the withdrawal process.

As an optional implementation, in the first aspect of the embodiments of the application, the operation that the plurality of OCT images are divided into four areas includes the following operation.

The OCT images are equally divided into four parts, and are sequentially classified into a uterine fundus area image, a uterine upper section area image, a uterine lower section area image, and a cervical area image according to an acquisition time sequence of each OCT image.

Or,
the OCT images are divided into four parts according to a certain proportion, and are sequentially classified into the uterine fundus area image, the uterine upper section area image, the uterine lower section area image, and the cervical area image according to the acquisition time sequence of each OCT image.

As an optional implementation, in the first aspect of the embodiments of the application, the image acquisition apparatus includes a light source, an optical splitter, and a detector. The operation that the image acquisition apparatus is started to obtain the plurality of OCT images includes the following operations.

The light source is started. Light emitted by the light source is split into reference light and signal light by the optical splitter.

The signal light is emitted to an image acquisition area through the probe, and the light reflected and scattered by the image acquisition area is returned to the detector to form a signal arm.

The reference light is directly emitted to the detector through an optical path having the same optical distance as the signal arm to form a reference arm.

The signal arm and the reference arm interfere with each other to form an interference optical signal, which is photoelectrically converted into an electrical signal through the detector.

The OCT images are obtained through analog-to-digital conversion and Fourier transform.

As an optional implementation, in the first aspect of the embodiments of the application, after the plurality of OCT images are obtained, the method further includes the following operations.

Image enhancement processing is performed on the OCT images. The image enhancement processing is to perform Red Green Blue (RGB) three-color mapping on the OCT images by using different pseudo-color palettes to obtain pseudo-color images of different tones.

A second aspect of the embodiments of the application discloses an electronic device, which includes a first driving unit, a second driving unit, an image acquisition unit, and an image receiving unit.

The first driving unit is configured to drive an OCT probe to be inserted into a uterine cavity through a cervical orifice until reaching a fundus of a uterus.

The second driving unit is configured to drive the withdrawal of the OCT probe.

The image acquisition unit is configured to start an image acquisition apparatus to obtain a plurality of OCT images.

The image receiving unit is configured to receive the plurality of OCT images and divide the plurality of OCT into four areas, namely a uterine fundus area, a uterine upper section area, a uterine lower section area, and a cervical area, each area having one or more OCT images.

A third aspect of the embodiments of the application discloses a detection system directly applying noninvasive OCT to an endometrium, which includes an OCT device, a catheter, a reproductive tract guide, a power mechanism, and an electronic device.

The OCT device is configured to acquire a plurality of OCT images, the OCT device includes an image acquisition apparatus and an OCT probe, the image acquisition apparatus being connected to the OCT probe through an optical path.

The catheter is configured to mount the OCT probe.

The reproductive tract guide is configured to connect the catheter.

The power mechanism is configured to drive the catheter to act through the reproductive tract guide.

The electronic device is configured to drive the OCT probe to be inserted into a uterine cavity through a cervical orifice until reaching a fundus of a uterus through the power mechanism, drive the withdrawal of the OCT probe through the power mechanism, and start the image acquisition apparatus to obtain a plurality of OCT images. The electronic device is further configured to receive the plurality of OCT images and divide the plurality of OCT images into four areas, namely a uterine fundus area, a uterine upper section area, a uterine lower section area, and a cervical area, each area having one or more OCT images.

A fourth aspect of the embodiments of the application discloses a computer-readable storage medium, in which a computer program is stored. The computer program causes a computer to perform the detection method directly applying the noninvasive OCT to the endometrium disclosed in the first aspect of the embodiments of the application.

A fifth aspect of the embodiments of the application discloses a computer program product. The computer program product runs on a computer to cause the computer to perform the detection method directly applying the noninvasive OCT to the endometrium disclosed in the first aspect of the embodiments of the application.

A sixth aspect of the embodiments of the application discloses an application publishing platform. The application publishing platform is configured to publish a computer program product. The computer program product runs on a computer to cause the computer to perform the detection method directly applying the noninvasive OCT to the endometrium disclosed in the first aspect of the embodiments of the application.

Compared with the related art, the embodiments of the application have the following beneficial effects.

In the embodiments of the application, the OCT probe is inserted into the uterine cavity through the cervical orifice until reaching the fundus of the uterus; the OCT probe is withdrawn, and the image acquisition apparatus is started at the same time to obtain the plurality of OCT images; and the plurality of OCT images are divided into four areas, namely the uterine fundus area, the uterine upper section area, the uterine lower section area, and the cervical area, each area having one or more OCT images, and the OCT image of each area is analyzed. It can be seen that, by implementing the embodiments of the application, each area may be analyzed according to the OCT image, a human body cannot be injured, and the detection is accurate.

### Brief Description of the Drawings

In order to explain the technical solutions in the embodiments of the application more clearly, the following will briefly introduce the accompanying drawings used in the description of the embodiments. It is apparent that the accompanying drawings described below are only some embodiments of the application. Other accompanying drawings may further be obtained by those of ordinary skill in the art according to these accompanying drawings without creative efforts.
Fig. 1 is a flowchart of a detection method directly applying noninvasive OCT to an endometrium disclosed in an embodiment of the application.
Fig. 2 is a schematic structural diagram of an electronic device disclosed in an embodiment of the application.

### Detailed Description of the Embodiments

The technical solutions in the embodiments of the application will be clearly and completely described in conjunction with the accompanying drawings in the embodiments of the application. It is apparent that the described embodiments are only a part of the embodiments of the application, and not all of them. All other embodiments obtained by those of ordinary skill in the art based on the embodiments of the application without creative efforts are within the scope of protection of the application.

It is to be noted that, terms "first", "second", "third", "fourth" and the like in the specification and claims of the application are used for distinguishing different objects rather than describing a specific sequence. Terms "include" and "have" and any variations thereof in the embodiments of the application are intended to cover non-exclusive inclusions. Exemplarily, it is not limited for processes, methods, systems, products or devices containing a series of steps or units to clearly list those steps or units, and other steps or units which are not clearly listed or are inherent to these processes, methods, products or devices may be included instead.

The embodiments of the application disclose a detection method and system directly applying noninvasive OCT to an endometrium, and an electronic device, which may analyze each area according to an OCT image, cannot injure a human body, and are accurate in detection, and are described in detail below in conjunction with the accompanying drawings.

### Embodiment 1

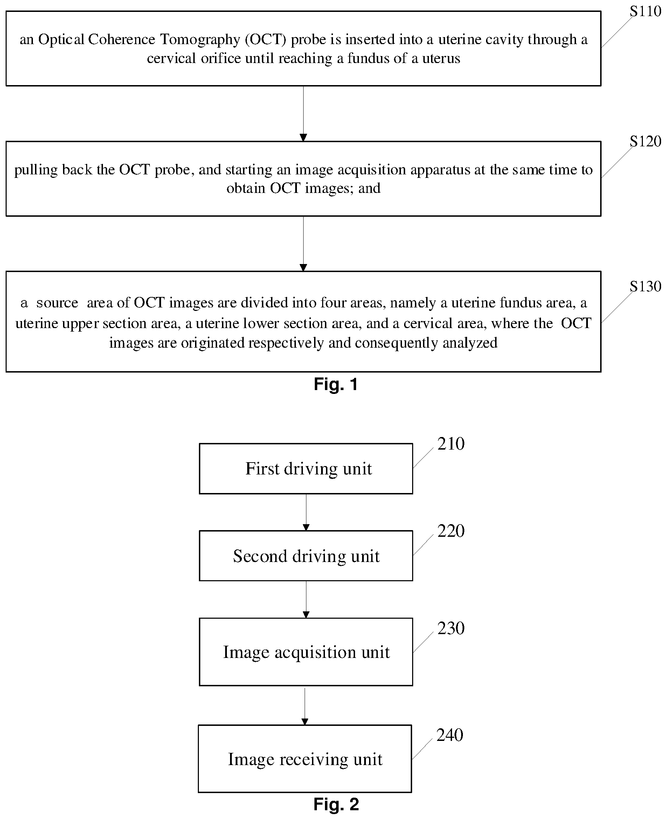

Referring to Fig. 1, Fig. 1 is a flowchart of a detection method directly applying noninvasive OCT to an endometrium disclosed in an embodiment of the application. As shown in Fig. 1, the detection method directly applying the noninvasive OCT to the endometrium includes the following operations.

At S110, an OCT probe is inserted into a uterine cavity through a cervical orifice until reaching a fundus of a uterus.

Before use, a device is connected to a power supply to turn on an electronic device connected to the OCT device. The electronic device may be a computer system, of course, or other terminal devices capable of controlling the OCT device to act or/and receive OCT images, such as a tablet computer, a cloud server, etc. Case information is newly created through the electronic device, and the personal information and the like of a related detected person may be entered. Of course, in some cases, the electronic device may also be integrated into the OCT device.

The electronic device is connected to the OCT device. For differentiation, the OCT device is classified into an image acquisition apparatus and the OCT probe. The OCT probe is mounted in a catheter, the OCT probe and a reflector are mounted in the catheter, and when light passes through the probe, the light is emitted by the reflector through a light emitting window. The probe may use an optical lens such as a cylindrical self-focusing lens (G-Lens), a front end of the self-focusing lens forms an inclined surface, a front end of the catheter is provided with the light emitting window, the inclined surface corresponds to the light emitting window, the inclined surface is provided with a light reflection film, and a back end of the self-focusing lens is fused to an optical fiber. A light reflection structure is the light reflection film, and the reflectivity of the light reflection film to the incident light may be greater than 99%. When the light emitted by the image acquisition apparatus is emitted to the probe through the optical fiber, the light is emitted to the external environment through the inclined surface and the light emitting window, that is, corresponding to a corresponding detection position.

In use, for accurate detection and ease of operation, the detected person empties a bladder and is placed in a bladder lithotomy position, a vulva and a vagina are routinely disinfected, and the uterine cavity is exposed with a speculum.

When the OCT device is used, image acquisition starts from the fundus of the uterus, so that the OCT probe needs to be pushed to the fundus of the uterus first. Exemplarily, a reproductive tract guide is connected to the catheter, the reproductive tract guide is manually or automatically controlled to drive the catheter to be inserted into a cervix through the cervical orifice until reaching the fundus of the uterus through the uterine cavity. In a manual manner, for example, a handle of the reproductive tract guide may be held to slowly insert the catheter into the uterine cavity through the cervical orifice. In an automatic manner, for example, the reproductive tract guide is controlled by a servo motor or the like to drive the catheter to be inserted into the uterine cavity through the cervical orifice.

In order to prevent the OCT probe from injuring the human body, in a better embodiment of the application, after being inserted into the cervix, the OCT probe slowly reaches the fundus of the uterus, preferably at a speed of no more than 0.3 cm/s.

In a preferred embodiment of the application, a length of the catheter outside the vagina when reaching the fundus of the uterus may also be recorded, or a length of the catheter extending into the uterus is determined, or a uterine length of the detected person is obtained in other manners for further use.

At S120, the OCT probe is withdrawn, and the image acquisition apparatus is started at the same time to obtain a plurality of OCT images.

The OCT probe is slowly taken, and the image acquisition apparatus is started to acquire the plurality of OCT images in a withdrawal process of the OCT probe.

As an implementation, the withdrawal process of the OCT probe is implemented at a constant speed, for example, the power mechanism drives the OCT probe to withdraw from the fundus of the uterus to the cervical orifice at the constant speed. When the OCT probe is withdrawn, the image acquisition apparatus is started to acquire the plurality of OCT images in the withdrawal process at a fixed frequency, namely a fixed time interval such as 10 ms. In this manner, a speed of withdrawal is preferably determined according to the number of the images to be acquired. Generally, the speed of withdrawal is preferably 0.1 to 0.3 cm/s, so that the number of the shot images may meet the needs, and at the same time, a balance between the time and efficiency is also achieved.

As another implementation, the OCT probe is driven to withdraw from the fundus of the uterus to the cervical orifice, and a position of movement of the OCT probe is acquired through a displacement sensor. When a distance between the positions of two times of movement is equal to a set distance (a plurality of set distances may be preset, one of which is selected as needed, or one is set before use as needed), the image acquisition apparatus is started to acquire the plurality of OCT images in the withdrawal process. In this manner, although the withdrawal may not be performed at the constant speed, generally, a movement speed of the OCT probe should enable the image acquisition apparatus to acquire the corresponding image at a set distance position, such as 30 mm.

In addition, another implementation is also provided, according to the weights of different image acquisition positions, that is, for an area that needs to be emphatically analyzed, the image acquisition frequency is higher, and the frequencies of other areas may be appropriately reduced. Exemplarily, when the uterine upper section area needs to be emphatically analyzed, the speed of withdrawal may be less than 0.1 cm/s at this stage. For example, if the withdrawal is implemented at the constant speed, and the speed of withdrawal is in the range of 0.1 to 0.3 cm/s, the speed of withdrawal may be selected to be 0.1 cm/s and 0.3 cm/s in other areas at this stage.

The actual length of each area may be obtained according to the total length and the conventional length ratio of each area. The uterine length may be obtained through S110. The conventional length may be obtained by obtaining the measurement data of people of different age groups, and then taking an average, so as to obtain the conventional length ratio of each area at each age group. For the detected person, the length of each area may be determined in conjunction with the conventional length ratio and the uterine length obtained in S110 according to the personal basic information filled in, and then the number or speed of image acquisition of each area may be limited as needed.

The process of determining the number of image acquisition of each area according to the weight may be implemented either by withdrawing the OCT probe at the constant speed and acquiring the images at the fixed time interval, or by achieving image acquisition by combining the displacement sensor with the set distance, which is not limited herein.

The image acquisition apparatus may include a light source, an optical splitter, a detector, etc. The operation that the image acquisition apparatus is started to obtain the plurality of OCT images includes: starting the light source. A manner of starting the light source may be implemented by the electronic device or may be implemented manually, and the light source may use a swept-frequency laser.

The light emitted by the light source is split into reference light and signal light by the optical splitter. The signal light is emitted to an image acquisition area through the probe, and the light reflected and scattered by the image acquisition area is returned to the detector to form a signal arm. The reference light is directly emitted to the detector through an optical path having the same optical distance as the signal arm to form a reference arm. The equal optical path may be achieved by an adjustable optical delay apparatus on the reference arm (also by adjusting a laser wavelength, a sweep frequency, and a polarization state), so that the optical paths of the two are equal by adjusting the optical delay apparatus, thereby generating an interference phenomenon.

The signal arm and the reference arm interfere with each other to form an interference optical signal, which is photoelectrically converted into an electrical signal through the detector. The OCT images are obtained through analog-to-digital conversion and Fourier transform.

Finally, through three-color mapping, a high-resolution tomography image inside a tissue may be obtained. If RGB three-color mapping (different pseudo-color palettes) is performed on intensity values of sampling points of the OCT image, pseudo-color images of different tones (such as slight red, slight purple, slight green, etc.) may be obtained.

At S130, the plurality of OCT images are divided into four areas, namely a uterine fundus area, a uterine upper section area, a uterine lower section area, and a cervical area, each area having one or more OCT images, and the OCT image of each area is analyzed.

In a preferred embodiment of the application, as a generalized uterine image, four areas, namely the uterine fundus area, the uterine upper section area, the uterine lower section area, and the cervical area, may be divided, each area may be divided according to the actual situation of the detected person, such as the above conventional length ratio, of course, in a case where the requirements for the results are not too strict, each area may also be equally divided.

Based on this, as an implementation, the OCT images are equally divided into four parts, and are sequentially classified into a uterine fundus area image, a uterine upper section area image, a uterine lower section area image, and a cervical area image according to an acquisition time sequence of each OCT image. The OCT image of each area may be called an OCT area image.

As another implementation, the OCT images are divided into four parts according to a certain proportion (such as the above conventional length ratio of different age groups or other proportional manners), and are sequentially classified into the uterine fundus area image, the uterine upper section area image, the uterine lower section area image, and the cervical area image according to the acquisition time sequence of each OCT image.

It is to be understood that the above division is the division of a circular transverse image for the uterus. Before and after the division of the area, these OCT images may also be integrated into a square longitudinal image. Through the preprocessing of the longitudinal image, images with acquisition defects caused by the OCT device in the transverse images may be deleted. For example, discontinuous parts are deleted through the continuity of an epithelial layer or a endometrial layer. In order to prevent accidental deletion, the number of the continuous parts in the transverse image may be set. Exemplarily, when there are more than five discontinuous images, the images are considered to have defects, and when the endometrium is analyzed, the images are abandoned.

In some other embodiments, the OCT area images may be analyzed by the electronic device or manually. During the analysis, for each area, a certain number of, such as five OCT area images, may be selected for analysis, of course, for areas with larger weights, some OCT images may also be selected as analysis objects.

Exemplarily, taking manual analysis as an example, the epithelial layer and the endometrial layer are mainly analyzed.

The regularity and continuity of the epithelial layer are analyzed. If an epithelial edge is regular and smooth, the pithelial layer is regular, and if the epithelial edge has a depression, wrinkle or bulge, the pithelial layer is irregular. When the epithelial edge is intact, the epithelial layer is continuous, and when the epithelial edge is broken and missing, the epithelial layer is discontinuous.

The sizes of glands, the cystic changes, whether a red dot area exists, and whether the endometrium has a defect are analyzed. The morphology of the glands, whether the glands are enlarged or increased, and the areas with cystic features of the endometrium are observed. When a pixel value (gray value) of the OCT image is within a certain range, such as 101 to 255, the area formed by these pixels is the red dot area. When there is a low signal area such as dark shadow or hole in the tissue of the endometrium, it indicates that the endometrium has the defect.

The above indicators may also be achieved by artificial intelligence, for example, by performing OCT image acquisition on different age groups and different health degrees of the uterus, training and testing OCT images of each age group with a model such as a neural network model based on the above indicators, and then analyzing the subsequent detected person according to the model.

Of course, the preprocessing of the above longitudinal image may also be implemented by artificial intelligence.

By implementing the embodiments of the application, each area may be analyzed according to the OCT image, the human body cannot be injured, and the detection is accurate.

### Embodiment 2

Referring to Fig. 2, Fig. 2 is a schematic structural diagram of an electronic device disclosed in an embodiment of the application. As shown in Fig. 2, the electronic device may be a computer system, of course, or other terminal devices capable of controlling an OCT device to act or/and receive OCT images, such as a tablet computer, a cloud server, etc. The electronic device may include: a memory, in which an executable program code is stored; and a processor coupled to the memory. The processor calls the executable program code stored in the memory.

As a virtual unit of the electronic device, the processor may include a first driving unit 210, a second driving unit 220, an image acquisition unit 230, and an image receiving unit 240.

The first driving unit 210 is configured to drive an OCT probe to be inserted into a uterine cavity through a cervical orifice until reaching a fundus of a uterus.

The second driving unit 220 is configured to drive the withdrawal of the OCT probe.

The image acquisition unit 230 is configured to start an image acquisition apparatus to obtain a plurality of OCT images.

The image receiving unit 240 is configured to receive the plurality of OCT images and divide the plurality of OCT into four areas, namely a uterine fundus area, a uterine upper section area, a uterine lower section area, and a cervical area, each area having one or more OCT images.

As an optional implementation, the first driving unit 210 may be configured to drive a reproductive tract guide to drive a catheter to be inserted into the uterine cavity through the cervical orifice until reaching the fundus of the uterus. The reproductive tract guide is connected to the catheter, and the OCT probe is mounted in the catheter. A speed of insertion into the uterine cavity is not more than 0.3 cm/s.

As an optional implementation, the second driving unit 220 may include a first withdrawal subunit and a first acquisition subunit.

The first withdrawal subunit is configured to start a power mechanism to drive the OCT probe to withdraw from the fundus of the uterus to the cervical orifice at a constant speed. A speed of withdrawal is 0.1 to 0.3 cm/s.

The first acquisition subunit is configured to withdraw the OCT probe, and start the image acquisition apparatus at the same time to acquire the plurality of OCT images in a withdrawal process at a fixed time interval.

As an optional implementation, the second driving unit 220 may include a second withdrawal subunit and a second acquisition subunit.

The second withdrawal subunit is configured to drive the OCT probe to withdraw from the fundus of the uterus to the cervical orifice, and acquire a position of movement of the OCT probe through a displacement sensor.

The second acquisition subunit is configured to start, when a distance between the positions of two times of movement is equal to a set distance, the image acquisition apparatus to acquire the plurality of OCT images in the withdrawal process.

As an optional embodiment, the image receiving unit 240 may include the following operations.

The OCT images are equally divided into four parts, and are sequentially classified into a uterine fundus area image, a uterine upper section area image, a uterine lower section area image, and a cervical area image according to an acquisition time sequence of each OCT image.

Or,
the OCT images are divided into four parts according to a certain proportion, and are sequentially classified into the uterine fundus area image, the uterine upper section area image, the uterine lower section area image, and the cervical area image according to the acquisition time sequence of each OCT image.

As an optional implementation, the image acquisition apparatus includes a light source, an optical splitter, and a detector. The image acquisition unit 230 may include a starting subunit, a first detection subunit, a second detection subunit, an interference subunit, and a processing subunit.

The starting subunit is configured to start a light source. Light emitted by the light source is split into reference light and signal light by the optical splitter.

The first detection subunit is configured to emit signal light to an image acquisition area through the probe, and return the light reflected and scattered by the image acquisition area to the detector to form a signal arm.

The second detection subunit is configured to directly emit the reference light to the detector through an optical path having the same optical distance as the signal arm to form a reference arm.

The interference subunit is configured to make the signal arm and the reference arm interfere with each other to form an interference optical signal, which is photoelectrically converted into an electrical signal through the detector.

The processing subunit is configured to obtain OCT images through analog-to-digital conversion and Fourier transform.

As an optional embodiment, the image acquisition unit 230 may further include an enhancement subunit.

The enhancement subunit is configured to perform image enhancement processing on the OCT images. The image enhancement processing is to perform RGB three-color mapping on the OCT images by using different pseudo-color palettes to obtain pseudo-color images of different tones.

### Embodiment 3

A detection system directly applying noninvasive OCT to an endometrium disclosed in Embodiment 3 of the application may include an OCT device, a catheter, a reproductive tract guide, a power mechanism, and an electronic device.

The OCT device is configured to acquire a plurality of OCT images, the OCT device includes an image acquisition apparatus and an OCT probe, the image acquisition apparatus being connected to the OCT probe through an optical path.

The catheter is configured to mount the OCT probe.

The reproductive tract guide is configured to connect the catheter.

The power mechanism is configured to drive the catheter to act through the reproductive tract guide.

The electronic device is configured to drive the OCT probe to be inserted into a uterine cavity through a cervical orifice until reaching a fundus of a uterus through the power mechanism, drive the withdrawal of the OCT probe through the power mechanism, and start the image acquisition apparatus to obtain a plurality of OCT images. The electronic device is further configured to receive the plurality of OCT images and divide the plurality of OCT images into four areas, namely a uterine fundus area, a uterine upper section area, a uterine lower section area, and a cervical area, each area having one or more OCT images.

The embodiments of the application disclose a computer-readable storage medium, in which a computer program is stored. The computer program causes a computer to perform part or all of the steps in the detection method directly applying the noninvasive OCT to the endometrium disclosed in Embodiment 1.

The embodiments of the application further disclose a computer program product. The computer program product runs on a computer to cause the computer to perform part or all of the steps in the detection method directly applying the noninvasive OCT to the endometrium disclosed in Embodiment 1.

The embodiments of the application further disclose an application publishing platform. The application publishing platform is configured to publish a computer program product. The computer program product runs on a computer to cause the computer to perform part or all of the steps in the detection method directly applying the noninvasive OCT to the endometrium disclosed in Embodiment 1.

In various embodiments of the application, it should be understood that, a magnitude of a sequence number of each process does not mean an execution sequence and the execution sequence of each process should be determined by its function and an internal logic and should not form any limit to an implementation process of the embodiments of the application.

The units described as separate components may or may not be physically separated. The components displayed as units may or may not be physical units, that is, the components may be located in one place, or may be distributed on the plurality of network units. Part or all of the units may be selected according to actual requirements to achieve the purposes of the solutions of this embodiment.

In addition, the functional units in the various embodiments of the application may be integrated into one processing unit, or each unit may exist alone physically, or two or more than two units may be integrated into one unit. The above integrated unit may be implemented in the form of hardware, or may be implemented in the form of a software functional unit.

If being implemented in form of the software functional unit and sold or used as an independent product, the integrated unit may be stored in a memory that may be obtained by the computer. Based on this understanding, the technical solutions of the application essentially or the parts that contribute to the conventional art, or all or part of the technical solutions may be embodied in the form of a software product. The computer software product is stored in the memory, including a plurality of requests for causing a computer device (which may be a personal computer, a server, or a network device, and the like, specifically, a processor in the computer device) to execute part or all of the steps of the method described in the various embodiments of the application.

In the embodiments provided by the application, it should be understood that "B corresponding to A" indicates that B is associated with A, and B may be determined according to A. However, it should also be understood that determining B according to A does not mean that B is determined only according to A, and B may also be determined according to A and/or other information.

Those of ordinary skill in the art should understand that part or all of the steps in the various methods of the embodiments may be implemented by related hardware instructed through a program, the program may be stored in a computer-readable storage medium. The storage medium includes: a Read-Only Memory (ROM), a Random Access Memory (RAM), a Programmable ROM (PROM), an Erasable PROM (EPROM), a One-time PROM (OTPROM), an Electrically EPROM (EEPROM), a Compact Disk Read-Only Memory (CD-ROM) or other optical disk storage, magnetic disk storage, magnetic tape storage, or any other computer-readable medium that can be configured to carry or store data.

The detection method and system directly applying the noninvasive OCT to the endometrium, and the detection device disclosed in the embodiments of the application are described above in detail. The principles and implementations of the application are described herein using specific examples, the foregoing description of the examples are only used to help the understanding of the method and core concept of the application. At the same time, for those of ordinary skill in the art, according to the concept of the application, there will be changes in the specific implementations and the application scope. In summary, the contents of the present description should not be construed as limiting the application.

## Claims

1. A detection method directly applying noninvasive Optical Coherence Tomography (OCT) to an endometrium, comprising:
inserting an OCT probe into a uterine cavity through a cervical orifice until reaching a fundus of a uterus;
withdrawing the OCT probe, and starting an image acquisition apparatus at the same time to obtain a plurality of OCT images; and
dividing the plurality of OCT images into four areas, namely a uterine fundus area, a uterine upper section area, a uterine lower section area, and a cervical area, each area having one or more OCT images, and analyzing the OCT image of each area.

2. The detection method directly applying the noninvasive OCT to the endometrium as claimed in claim 1, wherein the inserting an OCT probe into a uterine cavity through a cervical orifice until reaching a fundus of a uterus comprises:
connecting a catheter provided with the OCT probe by using a reproductive tract guide; and
manually or automatically controlling the reproductive tract guide to drive the catheter to be inserted into the uterine cavity through the cervical orifice until reaching the fundus of the uterus.

3. The detection method directly applying the noninvasive OCT to the endometrium as claimed in claim 2, wherein a speed of manual or automatic control is not greater than 0.3 cm/s.

4. The detection method directly applying the noninvasive OCT to the endometrium as claimed in claim 1, wherein the withdrawing the OCT probe, and starting an image acquisition apparatus at the same time to obtain a plurality of OCT images comprises:
starting a power mechanism to drive the OCT probe to withdraw from the fundus of the uterus to the cervical orifice at a constant speed, wherein a speed of withdrawal is 0.1 to 0.3 cm/s; and
withdrawing the OCT probe, and starting the image acquisition apparatus at the same time to acquire the plurality of OCT images in a withdrawal process at a fixed frequency.

5. The detection method directly applying the noninvasive OCT to the endometrium as claimed in claim 1, wherein the withdrawing the OCT probe, and starting an image acquisition apparatus at the same time to obtain a plurality of OCT images comprises:
driving the OCT probe to withdraw from the fundus of the uterus to the cervical orifice, and acquiring a position of movement of the OCT probe through a displacement sensor; and
when a distance between the positions of two times of movement is equal to a set distance, starting the image acquisition apparatus to acquire the plurality of OCT images in the withdrawal process.

6. The detection method directly applying the noninvasive OCT to the endometrium as claimed in claim 1, wherein the dividing the plurality of OCT images into four areas comprises:
equally dividing the OCT images into four parts, and sequentially classifying into a uterine fundus area image, a uterine upper section area image, a uterine lower section area image, and a cervical area image according to an acquisition time sequence of each OCT image;
or,
dividing the OCT images into four parts according to a certain proportion, and sequentially classifying into the uterine fundus area image, the uterine upper section area image, the uterine lower section area image, and the cervical area image according to the acquisition time sequence of each OCT image.

7. The detection method directly applying the noninvasive OCT to the endometrium as claimed in any one of claims 1 to 6, wherein the image acquisition apparatus comprises a light source, an optical splitter, and a detector; the starting an image acquisition apparatus to obtain a plurality of OCT images comprises:
starting the light source, wherein light emitted by the light source is split into reference light and signal light by the optical splitter;
emitting the signal light to an image acquisition area through the probe, and returning the light reflected and scattered by the image acquisition area to the detector to form a signal arm;
directly emitting the reference light to the detector through an optical path having the same optical distance as the signal arm to form a reference arm;
enabling the signal arm and the reference arm interfere with each other to form an interference optical signal, which is photoelectrically converted into an electrical signal through the detector; and
obtaining the OCT images through analog-to-digital conversion and Fourier transform.

8. The detection method directly applying the noninvasive OCT to the endometrium as claimed in any one of claims 1 to 6, after the plurality of OCT images are obtained, further comprising:
performing image enhancement processing on the OCT images, wherein the image enhancement processing is to perform Red Green Blue (RGB) three-color mapping on the OCT images by using different pseudo-color palettes to obtain pseudo-color images of different tones.

9. An electronic device, comprising:
a first driving unit, configured to drive an Optical Coherence Tomography (OCT) probe to be inserted into a uterine cavity through a cervical orifice until reaching a fundus of a uterus;
a second driving unit, configured to drive the withdrawal of the OCT probe;
an image acquisition unit, configured to start an image acquisition apparatus to obtain a plurality of OCT images; and
an image receiving unit, configured to receive the plurality of OCT images and divide the plurality of OCT into four areas, namely a uterine fundus area, a uterine upper section area, a uterine lower section area, and a cervical area, each area having one or more OCT images.

10. A detection system directly applying noninvasive Optical Coherence Tomography (OCT) to an endometrium, comprising:
an OCT device, configured to acquire a plurality of OCT images, the OCT device comprising an image acquisition apparatus and an OCT probe, the image acquisition apparatus being connected to the OCT probe through an optical path;
a catheter, configured to mount the OCT probe;
a reproductive tract guide, configured to connect the catheter;
a power mechanism, configured to drive the catheter to act through the reproductive tract guide; and
an electronic device, configured to drive the OCT probe to be inserted into a uterine cavity through a cervical orifice until reaching a fundus of a uterus through the power mechanism, drive the withdrawal of the OCT probe through the power mechanism, and start the image acquisition apparatus to obtain a plurality of OCT images; the electronic device is further configured to receive the plurality of OCT images and divide the plurality of OCT images into four areas, namely a uterine fundus area, a uterine upper section area, a uterine lower section area, and a cervical area, each area having one or more OCT images.
